# EUROPEAN PATENT APPLICATION

(11) **EP 3 960 128 A1**
(43) Date of publication of application: **02.03.2022**
(21) Application number: 21785739.0
(22) Date of filing: 18.01.2021
(51) Int. Cl.: A61F 2/24

(54) **HEART VALVE PROSTHESIS**

(30) Priority: 15.07.2020 CN 202010680119; 15.07.2020 CN 202021390480 U
(71) Applicant: Shanghai Trulive Medtech Co., Ltd, Shanghai 201206 (CN)
(72) Inventor: ZHAO, Jing, Pudong New District Shanghai 201306 (CN)
(74) Representative: Argyma
(86) International application number: PCT/CN2021/072481
(87) International publication number: WO 2022/012010

(57) **Abstract**

A heart valve prosthesis includes a stent and a valve. The stent includes a main body portion for supporting the valve and a frame portion for fixing the main body portion. The main body portion and the valve are fixed at the annulus via the frame portion to replace a native valve, thereby realizing functions of opening and closing a blood channel.

## Description

### BACKGROUND OF THE INVENTION

### Field of the Invention

The present invention relates to the technical field of medical instruments, and more specifically, to a heart valve prosthesis for implanting into a heart.

### Description of the Prior Art

The heart contains four heart cavities; the left atrium and left ventricle are located on the left side of the heart, and the right atrium and right ventricle are located on the right side of the heart. A ventricle inflow tract is formed between the atrium and the ventricle, the left ventricle and the aorta form the left ventricle outflow tract, and the right ventricle and the pulmonary artery form the right ventricle outflow tract. There is a valve with functions of a "one-way valve" at the left ventricle inflow tract and the left ventricle outflow tract to ensure the normal blood flow in the heart cavity. When there is a problem with the valve, cardiac hemodynamics change and the heart function is abnormal, resulting in a condition called a valvular heart disease.

The mitral regurgitation may cause myocardial remodeling, lead the ventricle to dilate progressively, and finally cause heart failure. The transcatheter mitral valve replacement (TMVR) adopts a method of intervening a catheter, wherein the prosthetic valve is compressed into a delivery system in vitro and transported to the human mitral valve annulus, and then the prosthetic valve is released and anchored at the mitral valve annulus to replace the primary valve. Compared with surgery, TMVR does not require extracorporeal circulation devices, has a small trauma, a quick recovery for the patients, and can significantly improve the hemodynamic index of the patients after operation.

Although the transcatheter mitral valve replacement technology develops rapidly, there are still some recognized challenges in the design of valves, for example:
1. The atrioventricular valve assembly has a complex structure, wherein if the subvalvular height of the prosthesis valve is too large, the structure and functions of the native heart may be affected, heart tissues such as the papillary muscle may be touched to cause abnormality while being prone to lead the left ventricle outflow tract obstruction (LVOTO), and adverse postoperative effects will be induced.
2. Compared with the aortic valve, the native valve annulus of the mitral valve have a larger diameter, and correspondingly the area of the valve leaflet of the prosthetic valve is larger.

The tricuspid valve also has the problem of the outflow tract obstruction; the existing valve stents are mostly of the single-layered structures, and the area of the valve leaflet corresponding to the stent of the single-layered structure is large, so that the larger the area of the valve leaflet is, the poorer the anti-fatigue performance is. At the same time, when the area of the valve leaflet is large, the size required for the stent is large correspondingly, and the diameter of the catheter for transporting the valve prosthesis will be also large, which increases the difficulty in transporting and the risk in the damage of the blood vessels.

### SUMMARY OF THE INVENTION

The present invention provides a heart valve prosthesis, which may solve the above drawbacks in the prior art.

The technical solution of the present invention is as follows:
A heart valve prosthesis includes a valve and a stent;
wherein the stent includes a main body portion for supporting the valve and a frame portion for fixing the main body portion at native valve annulus, and the main body portion is connected to the frame portion;
the frame portion includes an atrium segment deployed in an atrium, an annulus segment deployed at the annulus and a ventricle segment deployed in a ventricle; the ventricle segment includes a first ventricle segment located at a side close to the outflow tract, and a distance between the first ventricle segment and an axis of the frame portion is R1, wherein R1 decreases gradually in a linear or nonlinear manner in a direction from the atrium to the ventricle. The first ventricle segment is far away from the outflow tract gradually, and a longitudinal size of the frame portion abutting against the atrium and the annulus is small, so as to reduce the effects on the heart functions exerted by the valve prosthesis and prevent conduction block.

Since the valve is located in the main body portion of the stent, and a radial size of the main body portion is smaller as compared with the existing single-layered stent, so the size of the prosthetic valve leaflet is smaller and the anti-fatigue performance of the valve leaflet is improved; and since a radial size of the main body portion is smaller, an overall size of the valve prosthesis becomes smaller, which facilitates loading and transporting.

Preferably, an included angle between the tangent and the axial direction of the first ventricle segment is α, α being an acute angle; preferably, the included angle α ranges from 30° to 60° . If the included angle α is too small, a degree to which the first ventricle segment 2131 deviates from the outflow tract is too small, thereby increasing the risk in outflow tract obstruction; if the included angle α is too large, the resistance in pressing and holding will be increased, so the included angle α preferably ranges from 30° to 60° .

Preferably, the main body portion and the frame portion are of an internal-external nested structure, the main body portion is sleeved inside, and a distance between an axis of the main body portion and an axis of the frame portion is r, wherein r is greater than 0. The main body portion is fixed with the frame portion in an eccentric manner, and the main body portion is far away from one side of the outflow tract, thereby further reducing the risk in outflow tract obstruction. Studies have shown that if the prosthetic mitral valve may drive the blood to flow along a side wall of the ventricle, the blood may turn smoothly to create a large vortex, thereby ejecting the blood to the aorta and further flowing through the whole body. For the heart valve with the eccentric structure, as the valve leaflet is located on the stent main body and the stent main body is biased towards one side of the wall of the ventricle, the blood flow may tend to flow along the side wall of the ventricle, which is more beneficial to maintain a natural "blood flow vortex" for the left atrium, thereby promoting the recovery for the ventricle functions, especially for the vulnerable patients with severely-damaged heart conditions.

Preferably, the main body portion is far away from the outflow tract and abuts against the frame portion; more connection points may be generated when the above two abuts against each other for facilitating the fixed connection between the above two, so that the main body portion is as far away from the side of the outflow tract as possible, thereby reducing the risk in outflow tract obstruction.

Preferably, the frame portion and the main body portion are of a left-right parallel structure, wherein the frame portion is located a side close to the outflow tract, the main body portion is located outside the frame portion, and the frame portion abuts against a peripheral side of the main body portion and is connected to the same in a position where the two abuts against each other. With this structure, more connection points may be generated between the frame portion and the main body portion, so that the connection between the two may be more firm, and further the main body portion is as far away from the side of the outflow tract as possible, thereby further reducing the risk in outflow tract obstruction.

Preferably, the frame portion is of a non-enclosed structure. The non-enclosed structure may reduce the amount of materials used for the stent, and the non-enclosed structure enable the valve prosthesis to be easily pressed and held.

In a preferred embodiment, the frame portion is of an opening structure along an axial direction, and the frame portion is connected to a peripheral side of the main body portion at the opening. The non-enclosed structure may reduce the amount of materials used for the stent, so that the valve prosthesis is easy to press and hold and transport as the amount of the material is reduced.

Preferably, the stent further includes a fixing portion fixed with a delivery system, and the fixing portion is configured at an end of the main body portion or at an end of the frame portion. Connecting the fixing portion with the delivery system is used to load and release the valve prosthesis into and from the delivery system, and ensures that the valve prosthesis may not be displaced when being transported in the delivery system.

Preferably, the valve includes prosthetic valve leaflets, and the valve leaflets are disposed at the main body portion. One end of the valve leaflet is connected to the main body portion, and free ends of the valve leaflet are engaged with each other.

When the valve is in a working state, the native valve leaflets are replaced by the prosthetic valve leaflets to realize functions of opening and closing a blood channel.

Preferably, the stent is further provided with a skirt for sealing. The skirt is used for realizing sealing functions to ensure that a single channel of the blood is an outflow tract end flowing from an inflow tract end of the valve prosthesis to the valve prosthesis, which may effectively prevent the peripheral leakage and the regurgitation of the valve.

Compared with the conventional art, the present invention has the following beneficial effects:
First, in the present invention, the main body portion and the valve are fixed at native valve annulus of the inflow tract via the frame portion to replace a native valve, thereby realizing functions of opening and closing a blood channel, and at the side close to an outflow tract, a first ventricle segment of the frame portion is far away from the outflow tract and a longitudinal size of the frame portion abutting against the ventricle and the annulus is small, so that the main body portion is also as far away from the outflow tract as possible, which may reduce the effects on the heart functions exerted by the valve prosthesis, thereby preventing conduction block.

Second, since valve leaflets are located in the main body portion of the stent and a radial size of the main body portion is smaller as compared with the existing single-layered stent, a size of a prosthetic valve leaflet is smaller such that the anti-fatigue performance of the valve leaflet is improved; meanwhile, since the radial size of the main body portion is smaller, an overall size of the valve prosthesis becomes smaller and a pressing and holding space of the outflow tract is saved, so that a size required for delivery system is reduced, thereby facilitating loading and transporting.

Third, when there is a pre-set distance between the main body portion and the axis of the frame portion, i.e., in the eccentric structure, or when the main body portion and the frame portion are of a left-right connected structure, if the structure is used as the mitral valve, since the stent main body is biased towards one side of the wall of the ventricle, the blood flow may tend to flow along the side wall of the ventricle, which is more beneficial to maintain a natural "blood flow vortex" for the left atrium, thereby promoting the recovery for the ventricle functions, especially for the vulnerable patients with severely-damaged heart conditions.

Fourth, when the main body portion and the frame portion are of the left-right connected structure, the amount of the material for the stent is reduced; when the frame portion is of the non-enclosed structure, the amount of the material for the stent is further reduced, so that the pressing and holding, loading and transportation for the valve prosthesis may be facilitated as the amount of the material is reduced.

Certainly, any one product for implementing the present invention is unnecessary to achieve all the above advantages at the same time.

### BRIEF DESCRIPTION OF THE DRAWINGS

Fig. 1 is an overall structural schematic diagram of a stent according to Embodiment 1 of the present invention;
Fig. 2 is a structural schematic diagram of a frame portion according to Embodiment 1 of the present invention;
Fig. 3 is a structural schematic diagram of transporting a valve prosthesis into a heart according to Embodiment 1 of the present invention;
Fig. 4 is an illustrative perspective diagram of the stent according to Embodiment 1 of the present invention;
Fig. 5 is a structural schematic diagram of a main body portion according to Embodiment 1 of the present invention;
Fig. 6 is a top diagram of the main body portion according to Embodiment 1 of the present invention;
Fig. 7 is a top structural schematic diagram of the stent according to Embodiment 1 of the present invention;
Fig. 8 is another top structural schematic diagram of the stent according to Embodiment 1 of the present invention;
Fig. 9 is a structural schematic diagram of the stent and anchoring according to Embodiment 1 of the present invention;
Fig. 10 is a top structural schematic diagram of the stent and anchoring according to Embodiment 1 of the present invention;
Fig. 11 is a structural schematic diagram of the stent according to Embodiment 2 of the present invention;
Fig. 12 is an illustrative structural schematic diagram of the stent according to Embodiment 2 of the present invention;
Fig. 13 is a bottom diagram of the frame portion according to Embodiment 3 of the present invention;
Fig. 14 is an illustrative structural schematic diagram of the stent according to Embodiment 3 of the present invention.

Reference for numerals: stent-100; main body portion-110; frame portion-210; atrium segment-211; annulus segment-212; ventricle segment-213; first ventricle segment-2131; second ventricle segment-2132; inflow segment-Ill; outflow segment-113; transition segment-112; hanging tab-114; fixing tab-214.

### DESCRIPTION OF THE PREFERRED EMBODIMENTS

The present invention provides a heart valve prosthesis, which includes a valve and a stent for fixing the valve at native valve annulus, wherein the stent includes a main body portion for supporting the valve and a frame portion for fixing the main body portion, and the main body portion is connected to the frame portion, and wherein at a side close to the outflow tract, a longitudinal size of the frame portion abutting against the atrium and the annulus is small, so that the effects on the heart functions exerted by the valve prosthesis may be reduced when the first ventricle segment extends towards the main body portion, thereby preventing conduction block.

The heart valve prosthesis of the present invention is suitable for the mitral valve and the tricuspid valve. As mentioned here, when the valve prosthesis is the mitral valve, the valve prosthesis is fixed at the native valve annulus of the left ventricle inflow tract, and then the term "outflow tract" refers to the left ventricle outflow tract. When the valve prosthesis is the tricuspid valve, the valve prosthesis is fixed at the native valve annulus of the right ventricle inflow tract, and then the term "outflow tract" refers to the right ventricle outflow tract.

The present invention will be further described with the mitral valve as an example.

In order to describe the structural characteristics of the present invention more clearly, the terms "proximal end" and "distal end" are used as orientation words, wherein "proximal end" means an end close to the tip of the heart during surgery, and "distal end" means an end away from the tip of the heart.

As mentioned here, "longitudinal height" refers to a size of a component in an axial direction of the valve prosthesis. As mentioned here, "valve prosthesis" and "heart valve prosthesis" have the same meaning. As mentioned here, when used alone, "valve" refers to a plurality of prosthetic valve leaflets fixed circumferentially along the main body portion. As mentioned here, "left ventricle outflow tract" has the same meaning as LVOT.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The implant provided by the present invention will be further described in combination with drawings and specific embodiments. It should be noted that the drawings are all in a simplified form and both use non-precise proportions, and are only for convenience and clarity to assist the purpose of the embodiments of the invention. It should be understood that these embodiments are only used for illustrating the present invention, but not for the limitation of the scope of the present invention. In practical application, the improvement and adjustment made by those skilled in the art according to the present invention still belong to the scope of protection of the present invention.

### Embodiment 1

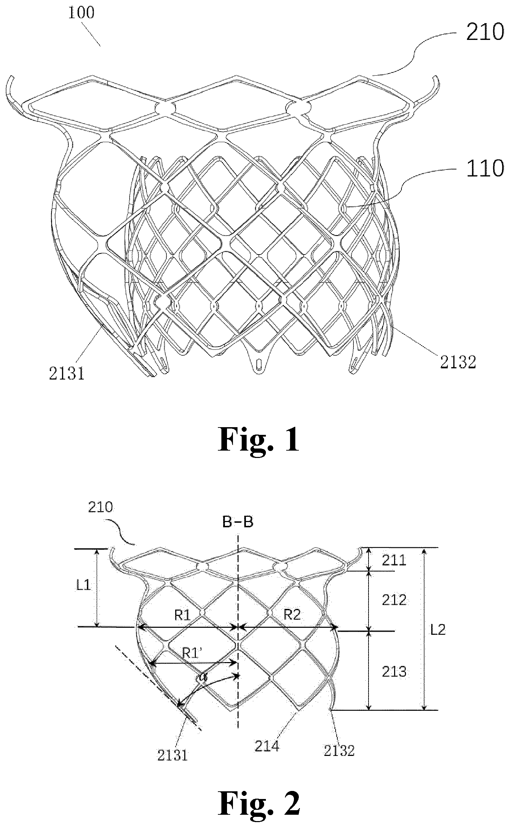

The present embodiment provides a mitral heart valve prosthesis. With reference to Figs. 1 to 10, a view of the valve prosthesis of the present embodiment is shown. The valve prosthesis includes a valve and a stent 100.

The stent 100 includes a main body portion 110 for supporting the valve and a frame portion 210 for fixing the main body portion 110, and the main body portion 110 is connected to the frame portion 210. The frame portion 210 includes an atrium segment 211 deployed in an atrium, an annulus segment 212 deployed at the annulus and a ventricle segment 213 deployed in a ventricle; the annulus segment 212 is located between the atrium segment 211 and the ventricle segment 213. The ventricle segment 213 includes a first ventricle segment 2131 and a second ventricle segment 2132, the first ventricle segment 2131 is located on a side closed to a left ventricle outflow tract, and the second ventricle segment 2132 is located on a side far away from the left ventricle outflow tract.

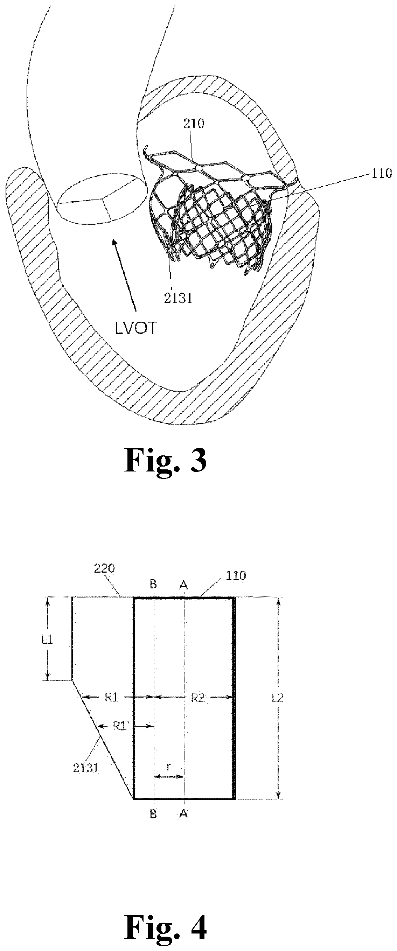

At the side close to the left ventricle outflow tract, the atrium segment 211 and the annulus segment 212 abut against the atrium and the annulus respectively, with a corresponding height L1, as shown in Fig. 2 and Fig. 4. At the side far away from the left ventricle outflow tract, the frame portion 210 has a height L2, wherein L1 is smaller than L2; and the first ventricle segment 2131 is configured to extend away from the left ventricle outflow tract, so as to reduce the effects on heart functions exerted by the valve prosthesis, thereby preventing conduction block.

With reference to Fig. 3, a native valve is replaced by fixing the main body portion 110 and the valve at the annulus of the left ventricle inflow tract with the frame portion 210. At one side of the left ventricle outflow tract, a longitudinal size of the frame portion 210 abutting against the atrium and the annulus is small, and the first ventricle segment 2131 of the frame portion 210 is far away from the left ventricle outflow tract, so that the effects on the heart functions exerted by the valve prosthesis is reduced, thereby preventing conduction block. At the same time, in the present embodiment, the valve leaflet are located in the main body portion of the stent, and a radial size of the main body portion is smaller as compared with the single-layered stent, so the size of the prosthetic valve leaflet is smaller and the anti-fatigue performance of the valve leaflet is improved. And, since the radial size of the main body portion is smaller, the overall size of the valve prosthesis becomes smaller, thereby facilitating loading and transporting.

With reference to Figs. 2 and 4, a distance between the first ventricle segment 2131 and an axis B-B of the frame portion 210 is R1, and a distance between the second ventricle segment 2132 and the axis B-B of the frame portion 210 is R2. In an embodiment, R1 decreases gradually from the atrium to the ventricle in a linear manner while R2 remaining unchanged, and then the first ventricle segment 2131 is a slope. Naturally, in other embodiments, R1 may also decrease gradually in a non-linear manner; for example then the first ventricle segment 2131 is an arc-shaped surface, thereby also reducing the risk in blockade of the left ventricle outflow tract. When the first ventricle segment 2131 is the arc-shaped surface, R1 refers a distance from the tangent of a predetermined point in the arc-shaped surface to the axis.

An included angle between the tangent and the axial direction of the first ventricle segment 2131 is a, a being an acute angle. If the included angle α is too small, a degree to which the first ventricle segment 2131 deviates from the outflow tract is too small, thereby increasing the risk in outflow tract obstruction; if the included angle α is too large, the resistance in pressing and holding will be increased, so the included angle α preferably ranges from 30° to 60°.

In the present embodiment, the main body portion 110 and the frame portion 210 are of an internal-external nested structure, the main body portion 110 is sleeved inside and the frame portion 210 is sleeved outside; a distance between an axis A-A of the main body portion 110 and the axis B-B of the frame portion 210 is r, wherein r is greater than 0, as shown in Fig. 4. In other words, an eccentric design is configured between the main body portion 110 and the frame portion 210, and the main body portion 110 deviates away from the left ventricle outflow tract, thereby further reducing the risk in left ventricle outflow tract obstruction.

The size of r determines the degree of eccentricity of the main body portion 110; the smaller the r is, the smaller the degree of eccentricity is; the greater the r is, the greater the degree of eccentricity of the main body portion 110 is. Preferably, in the present embodiment, the main body portion 110 is far away from the left ventricle outflow tract and abuts against the frame portion 210, as shown in Figs. 1, 3, 4, and 9. At this time, the r is the greatest, and a peripheral side of the main body portion 110 abuts against the frame portion 210. More connection points may be generated when the peripheral side of the main body portion 110 abuts against the frame portion 210 for facilitating the fixed connection between the above two, so that the main body portion is as far away from the side of the outflow tract as possible, thereby reducing the risk in outflow tract obstruction.

Meanwhile, studies have shown that if the prosthetic mitral valve may drive the blood to flow along a side wall of the ventricle, the blood may turn smoothly to create a large vortex, thereby ejecting the blood to the aorta and further to flow through the whole body. For the heart valve with the eccentric structure, as the valve leaflet are located on the stent main body and the stent main body is biased towards one side of the wall of the ventricle, the blood flow may tend to flow along the side wall of the ventricle, which is more beneficial to maintain a natural "blood flow vortex" for the left atrium, thereby promoting the recovery for the ventricle functions, especially for the vulnerable patients with severely-damaged heart conditions.

In the present embodiment, the connection between the frame portion 210 and the main body portion 110 may be realized by way of riveting, welding, snapping and stitching, or by way of gluing with adhesives, wherein relative displacement between the main body portion 110 and the frame portion 210 should be ensured not to occur. Specific connection ways may be selected according to the material and molding methods of the frame portion 210 and the main body portion 110, which is not limited here.

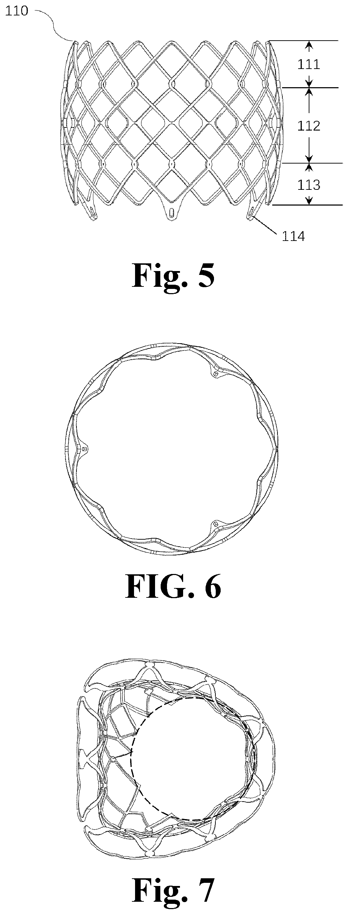

As shown in Fig. 5, a structural diagram of the main body portion 110 of the present embodiment is shown. The main body portion 110 includes an inflow segment 111, an outflow segment 113, and a transition segment 112 located between the inflow segment and the outflow segment. The main body portion 110 is an elliptical cylinder. Naturally, in other embodiments, the main body portion 110 may also be a cylinder, as shown in Fig. 7, or a cone, or an assembly structure of the cylinder and the cone. A cross-section of the main body portion 110 may be of a circle, or a circle-like shape, a D-shape, a flower shape or other irregular shapes. The circle-like shape refers to a hexagon and a polygon with more than six sides, with a nature similar to the circle; the flower shape is shown in Fig. 6.

The main body portion 110 is configured to be a mesh structure with a plurality of rows of constituent units. In the present embodiment, the constituent unit is of a diamond structure, which has the advantages of simple molding process and of forming a more flat diamond mesh surface. Naturally, in other embodiments, the constituent unit may also be a mesh unit that can be formed into an enclosed shape such as a triangle, a quadrate, a pentagon, a circle-like shape and a droplet shape, and the quadrate includes a rectangle as well as a square. The shape and size of the constituent unit and the number of rows of the constituent unit should be selected according to the material, process and the like of the main body portion.

The main body portion 110 may be selected from a self-expandable material and a plastic deformable material such as a balloon-expandable material, or may be a shape memory alloy that may respond to temperature changes to convert between a compressed state and an expanded state. Specifically, the main body portion 110 may adopt such as nitinol, titanium alloy, cobalt chromium alloy, MP35n, 316 stainless steel, L605, Phynox/Elgiloy and platinum chromium, or may be prepared by other bio-compatible metals known by those skilled in the art. Preferably, the main body portion 110 of the present embodiment is prepared by cutting a nickel-titanium alloy pipe, and the process is more mature and reliable, wherein an outer diameter of the pipe is 5mm to 15mm, the outer diameter of the pipe is a size in the natural state, and the shaped diameter size should be selected according to actual needs.

With continuous reference to Fig. 2, the frame portion 210 is an assembly structure of the cylinder, wherein the radial size of the atrium segment 211 is large, the ventricle segment 213 is of an asymmetric structure, and a cross-section of the frame portion 210 is the circle and the D-shape. Naturally, in other embodiments, the frame portion 210 may also be a cone, or a combination of the cylinder and the cone; the cross-section of the frame portion 210 may also have the circle-like shape, the D-shape, the flower shape or other irregular shapes, or a combination thereof.

The frame portion 210 is configured to be the mesh structure with a plurality of rows of constituent units, and in the present embodiment, the constituent unit is of a diamond structure. Naturally, in other embodiments, the constituent unit may also be a mesh unit that can be formed into the enclosed shape such as a triangle, a diamond, a pentagon, a circle-like shape and a droplet shape. The shape and size of the constituent unit and the number of rows of the constituent unit should be selected according to the material, process and the like of the main body portion.

The frame portion 210 may adopt such as nitinol, titanium alloy, cobalt chromium alloy, MP35n, 316 stainless steel, L605, Phynox/Elgiloy and platinum chromium, or may be prepared by other bio-compatible metals known by those skilled in the art. Optionally, the frame portion also includes an elastic deformable material or a plastic deformable material such as a balloon-expandable material, or may be a shape memory alloy that may respond to temperature changes to convert between a compressed state and an expanded state. Preferably, the frame portion 210 of the present embodiment is made by weaving the nickel-titanium alloy; in other optional embodiments, the frame portion 210 may be prepared by a material with a lower stiffness. In this way, the effects of the valve prosthesis on the heart functions may be further reduced.

Further, the stent 100 further includes a fixing portion fixed to a delivery system, and the fixing portion is connected to the delivery system, e.g., connected to a delivery sheath, so as to ensure that the relative position of the valve prosthesis and the delivery system remains unchanged when the valve prosthesis is loaded into the delivery system, the valve prosthesis is released and removed from the delivery system and the valve prosthesis is transported in vitro. With reference to Fig. 5, in the present embodiment, the fixing portion is a hanging tab 114 disposed at a proximal end of the outflow segment 113, and the hanging tab 114 is used to be connected to the delivery system. Naturally, in other embodiments, the fixing portion may also be configured at a distal end of the inflow segment 111, or at a proximal end of the outflow segment 113 and the distal end of the inflow segment 111 simultaneously.

In another optional embodiment, the fixing portion further includes a fixing tab 214 configured at the proximal end of the ventricle segment 213, as shown in Fig. 2. Naturally, in other alternative embodiments, the fixing portion may also be disposed at a distal end of the atrium segment 211, or at a proximal end of the ventricle segment 213 and the distal end of the atrium segment 211 simultaneously. The fixing portion is connected to the delivery system; therefore, the position and the number for configuring the fixing portion should be selected according to the type of the delivery system and the implantation manner, which is not limited here.

In the present embodiment, the valve prosthesis is a mitral valve prosthesis, and the valve has at least two valve leaflets; one end of the valve leaflet is directly or indirectly connected to the main body portion 110, and free ends of the valve leaflet are engaged with each other. When in a working state, the native valve leaflet is replaced by the prosthetic valve leaflet to realize functions of opening and closing a blood channel. The valve leaflet may adopt animal pericardium or other bio-compatible polymeric materials, such as PET or PTFE. The size and material may be selected according to different application scenarios.

In the present embodiment, the stent 100 is further provided with a skirt for sealing. The skirt is used for realizing sealing to ensure that a single channel of the blood is an outflow tract end flowing from an inflow tract end of the valve prosthesis to the valve prosthesis, thereby effectively preventing the peripheral leakage and the regurgitation of the valve. The skirt may be only disposed on the main body portion 110, or only disposed on the frame portion 210, or disposed on the main body portion 110 and the frame portion 210 simultaneously; the skirt may be disposed with a single side or both sides. When the skirt is disposed above a ring-shaped region formed by the main body portion 110 and the frame portion 210, the ring-shaped region is prevented from forming the thrombosis.

The material of the skirt is the animal pericardium such as porcine pericardium and bovine pericardium or other bio-compatible polymeric materials such as PET and PTFE. The region, the area and the material where the skirt is disposed should be configured according to actual clinical needs, which is not limited here.

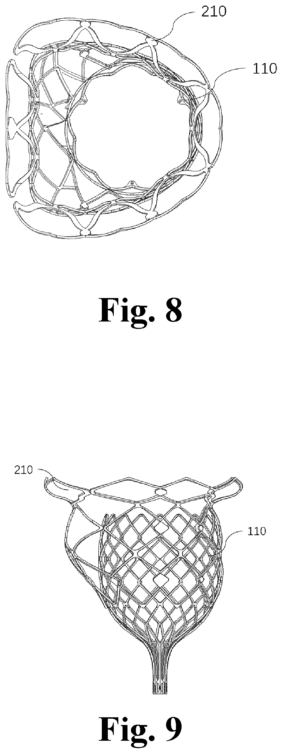

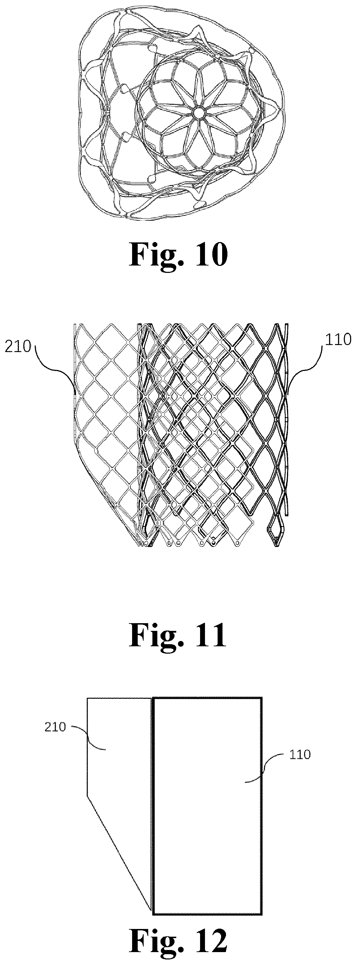

In the present embodiment, the anchoring form for the valve prosthesis is not limited, and a flange may be disposed on the stent 100, as shown in Figs. 7 and 8, wherein the anchoring form of Oversize is used; anchoring structures such as stabs and claws may also be disposed on the stent 100 for grabbing the native tissue, as shown in Figs. 1 and 3; the way in which the tether is anchored to the cardiac muscle wall is also used for fixing, or a combination of anchoring ways may also be used for anchoring. As shown in Figs. 9 and 10, the valve prosthesis adopts a combination of using the flange and the tether for fixing, the atrium segment 211 of the frame portion 210 is provided with the flange, and the outflow segment 113 of the main body portion 110 is connected to the tether for being anchored to the cardiac muscle wall by the tether. Specific anchoring way may be selected according to actual clinical needs.

### Embodiment 2

The present embodiment provides a valve prosthesis, which is an improvement made based on Embodiment 1, wherein the frame portion 210 and the main body portion 110 are of the left-right parallel structure.

With reference to Fig. 11, an overall structural diagram of the stent 100 of the present embodiment is shown, and Fig. 12 is an illustrative perspective diagram of the stent 100. The main body portion 110 is a cylinder, the main body portion 110 is located outside the frame portion 210, and a peripheral side of the frame portion 210 and a peripheral side of the main body portion 110 abut against each other and are connected with each other at a position where the two abut against each other. With this structure, more connection points may be generated by the frame portion 210 and the main body portion 110, so that a contact area of the frame portion 210 and the main body portion 110 is larger, so the connection of the above two becomes more firm; further, the main body portion 110 may be as far away from the left ventricle outflow tract as possible. Compared with the internal-external nested structure, the left-right connected structure may reduce the amount of material used for the frame portion 210, thereby facilitating pressing and holding.

When the valve prosthesis of the present embodiment is implanted, the frame portion 210 should be configured at a pre-set position close to the left ventricle outflow tract.

### Embodiment 3

The present embodiment provides a valve prosthesis, which is an improvement made based on Embodiment 1 or Embodiment 2, wherein the frame portion 210 is configured to be of a non-enclosed structure.

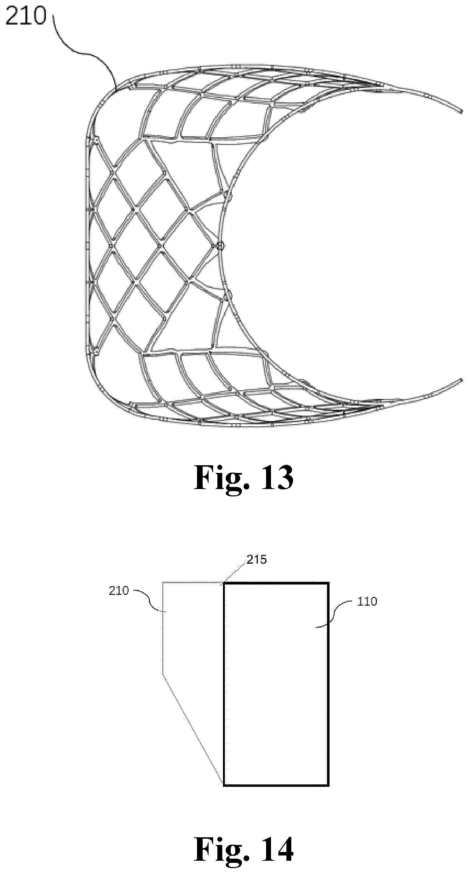

As shown in Fig. 13, a top view of the frame portion 210 is shown, and the frame portion 210 is of the non-enclosed structure with a cross-section of a semi-circle or a C-shape. The non-enclosed structure may further save the amount of material used for the frame portion 210, so that the valve prosthesis is easier to press, hold and transport.

As shown in Fig. 14, an illustrative perspective view of the stent of the present embodiment is shown, and the frame portion 210 and the main body portion 110 are of the left-right parallel structure, wherein the frame portion 210 is of an opening structure along an axial direction, and the frame portion 210 is connected to a peripheral side of the main body portion 110 at the opening.

Each embodiment in the specification is described in a progressive manner, the emphasis of each embodiment is put on explaining the difference from other embodiments and the same or similar parts of each embodiment may refer to each other. The above description is only a description of the better embodiment of the present invention, not any limitation of the scope of the present invention. Any changes or modifications made by those skilled in the art of the present invention in accordance with the above-mentioned disclosures shall fall within the scope of protection of the claims. It is obvious that various modifications and changes can be made to the content of the specification. The present invention selects and specifically describe the embodiments with the purpose of better explain the principle and practical use of the present invention, such that a person skilled in the art can well utilize the present invention. The present invention is merely limited by the appended claims and the scope and equivalents thereof.

## Claims

1. A heart valve prosthesis, comprising a valve and a stent;
wherein the stent comprises a main body portion for supporting the valve and a frame portion for fixing the main body portion at a native valve annulus, and the main body portion is connected to the frame portion; the frame portion comprises an atrium segment deployed in an atrium, an annulus segment deployed at the annulus and a ventricle segment deployed in a ventricle; the ventricle segment comprises a first ventricle segment located close to a side of the an outflow tract, and a distance between the first ventricle segment and an axis of the frame portion is R1, wherein R1 decreases gradually in a linear or nonlinear manner in a direction from the atrium to the ventricle.

2. The heart valve prosthesis according to claim 1, wherein an included angle between the tangent and the axial direction of the first ventricle segment is α , and the included angle α ranges from 30° to 60° .

3. The heart valve prosthesis according to claim 1, wherein the main body portion and the frame portion are of an internal-external nested structure, the main body portion is sleeved inside, and a distance between an axis of the main body portion and an axis of the frame portion is r, wherein r is greater than 0.

4. The heart valve prosthesis according to claim 3, wherein the main body portion is far away from the outflow tract and abuts against the frame portion.

5. The heart valve prosthesis according to claim 1, wherein the frame portion and the main body portion are of a left-right parallel structure.

6. The heart valve prosthesis according to any one of claims 1 to 5, wherein the frame portion is of a non-enclosed structure.

7. The heart valve prosthesis according to claim 6, wherein the frame portion is of an opening structure along an axial direction, and the frame portion is connected to a peripheral side of the main body portion at the opening.

8. The heart valve prosthesis according to claim 1, wherein the stent further comprises a fixing portion fixed with a delivery system, and the fixing portion is configured at an end of the main body portion or at an end of the frame portion.

9. The heart valve prosthesis according claims 1, wherein the valve comprises prosthetic valve leaflets, and the valve leaflets is arranged at the main body portion.

10. The heart valve prosthesis according to claims 1, wherein the stent is further provided with a skirt for sealing.
